# EUROPEAN PATENT APPLICATION

(11) **EP 3 299 035 A1**
(43) Date of publication of application: **28.03.2018**
(21) Application number: 17191691.9
(22) Date of filing: 18.09.2017
(51) Int. Cl.: A61L 2/20, B65B 55/10, A61L 2/10

(54) **A HYDROGEN PEROXIDE SUPPLY SYSTEM, AND A METHOD FOR PROVIDING A FLOW OF HYDROGEN PEROXIDE**

(30) Priority: 22.09.2016 EP 16190210
(71) Applicant: Tetra Laval Holdings & Finance S.A., 1009 Pully (CH)
(72) Inventor: RUNNBERG, Bo, 273 98 Smedstorp (SE)
(74) Representative: Tetra Pak - Patent Attorneys SE

(57) **Abstract**

A system (100) for hydrogen peroxide treatment is provided. The system comprises a supply (110) of hydrogen peroxide, a supply (120) of water, and an intermediate storage (130) for hydrogen peroxide being in fluid communication with the supply (110) of hydrogen peroxide and the supply (120) of water, wherein the system (100) further comprises a control unit (140) being connected to said supply (110) of hydrogen peroxide and the supply (120) of water for controlling the concentration of hydrogen peroxide in the intermediate storage (130).

## Description

### Technical field

The present solution relates to manufacturing of packages such as carton based packages for liquid food, and in particular to a hydrogen peroxide supply system for sterilization purposes during such manufacturing.

### Background

It is commonly known to use a carton based packaging material to form product containers, such as containers for enclosing and storing liquid food.

In order to ensure the required quality of the final package, e.g. in terms of food safety and integrity, the packaging material may comprise different layers. As an example, a packaging material may comprise a core material layer with at least one decorative layer applied on one side thereof making up the outer surface of the final package, and a polymeric composition or layer on the opposite or inner side. The polymeric composition may in some cases be provided with a protective film such as aluminum; the polymeric composition thus normally also includes an outer, or distal layer being in contact with the product intended to be contained in the final package.

Typically the packaging material is formed into semi-finished packages before they are filled with its desired content. Especially for food content it is required to sterilize the material of the package prior to filling. For such sterilization it is common to spray a gas mixture of hydrogen peroxide and air into the semi-finished package before any final content is introduced. The hot gas mixture will condense at the inner surface of the semi-finished package to form a thin liquid layer. This thin layer of sterilizing agent is then exposed to UV light for killing any microorganisms present inside the semi-finished package, and finally the remaining hydrogen peroxide will be vented before filling and sealing of the package is performed.

The hydrogen peroxide used for sterilizing is diluted to the desired concentration and is stored in a tank, and an operator is responsible for the correct concentration of the final gas mixture entering the semi-finished packages. However, it has been proven that once diluted and stored in a tank, the breakdown ratio of the hydrogen peroxide will increase. Due to this fact the ready-to-use hydrogen peroxide must be changed at machine stop, e.g. after nightly pauses in manufacturing. Additionally the continuous breakdown of hydrogen peroxide results in a difficulty to ensure the correct concentration of the gas mixture used for sterilization.

In view of this, it would be desired to have an improved system and method for hydrogen peroxide treatment in order to at least partly overcoming the disadvantages of prior art solutions.

### Summary

An object of the present solution is to solve the above-mentioned problems.

According to a first aspect, a system for hydrogen peroxide treatment is provided. The system comprises a supply of hydrogen peroxide, a supply of water, and an intermediate storage for hydrogen peroxide being in fluid communication with the supply of hydrogen peroxide and the supply of water, wherein the system further comprises a control unit being connected to said supply of hydrogen peroxide and the supply of water for controlling the concentration of hydrogen peroxide in the intermediate storage.

In an embodiment, supply of hydrogen peroxide comprises a first pump and said supply of water comprises a second pump, and wherein said control unit is connected to said pumps. In an alternative embodiment the supply of water may be a pressurized supply, whereby the need for a water supply pump is omitted.

The intermediate storage may comprise a level sensor for determining the fill level of said intermediate storage, and the control unit may be configured to control the supply of hydrogen peroxide and/or the supply of water based on the detected fill level. Accordingly, unintentional overflow of the intermediate storage is prevented.

The system may further comprise a sensor for detecting the concentration of hydrogen peroxide downstream the intermediate storage, and the control unit may be configured to control the supply of hydrogen peroxide and/or the supply of water based on the detected fill level. An accurate control of the actual concentration is thus accomplished, resulting in an improved quality of the sterilization process.

The sensor for detecting the concentration of hydrogen peroxide may be arranged in fluid communication with an outlet of the intermediate storage.

In an embodiment, the intermediate storage comprises a third pump for feeding diluted hydrogen peroxide from said intermediate storage to a downstream evaporator of a filling machine.

The system may further comprise mixing means arranged downstream the intermediate storage. The mixing means may be a recirculation line being provided with a flow restriction device arranged to recirculate at least some of the diluted hydrogen peroxide exiting the intermediate storage back to said intermediate storage. In another embodiment, the mixing means is a static mixer.

According to a second aspect, a method for providing a flow of hydrogen peroxide is provided. The method comprises providing a first feed of hydrogen peroxide to an intermediate storage, providing a second feed of water to said intermediate storage, and discharging diluted hydrogen peroxide from said intermediate storage. The method further comprises controlling the feeding rate of said first and second feeds in order to maintain a desired concentration of hydrogen peroxide in the discharge flow from said intermediate storage.

### Short description of the drawings

Fig. 1 illustrates an exemplary form, fill and seal packaging machine that includes a system for hydrogen peroxide treatment embodying the principles of the present invention.
Fig. 2 is a schematic view of a system for hydrogen peroxide treatment according to an embodiment.
Fig. 3 is a schematic view of a system for hydrogen peroxide treatment according to a further embodiment.
Fig. 4 is a schematic view of a method according to an embodiment.

### Detailed description

While the present solution is susceptible of embodiments in various forms, there is shown in the drawings and will hereinafter be described as presently preferred embodiments with the understanding that the present disclosure is to be considered an example embodiment of the solution and is not intended to limit the solution to the specific embodiments illustrated.

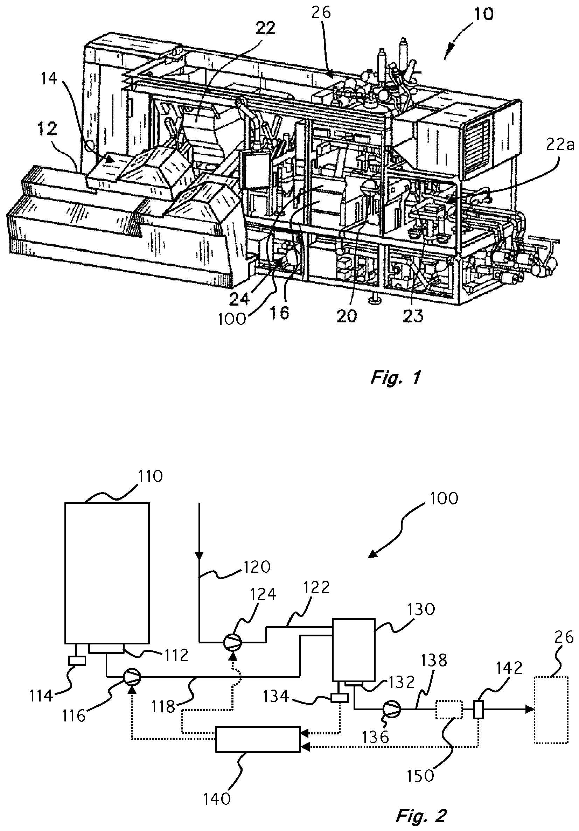

Referring now to Fig. 1 there is shown an exemplary form, fill and seal packaging machine 10 embodying the principles of the present solution. A conventional form, fill and seal packaging machine 10 includes a carton magazine 12 for storing flat, folded, carton blanks, a carton erection station 14 and a bottom forming and sealing station 22. The machine 10 further includes a sterilization station 16 for sterilizing the cartons and further includes a filling station 20 at which the cartons are filled with product and a top sealing station 22a at which the top panels of the cartons are pre-folded and subsequently sealed to one another. The cartons are then off loaded from the form, fill and seal packaging machine 10.

The sterilization station 16 is positioned between the bottom forming and sealing station 22 and the filling station 20. The sterilization station 16 can include one or more ultraviolet energy generating devices 24, and a hydrogen peroxide vapor generating system 26. The hydrogen peroxide vapor generating system 26 receives liquid hydrogen peroxide from a hydrogen peroxide supply system 100.

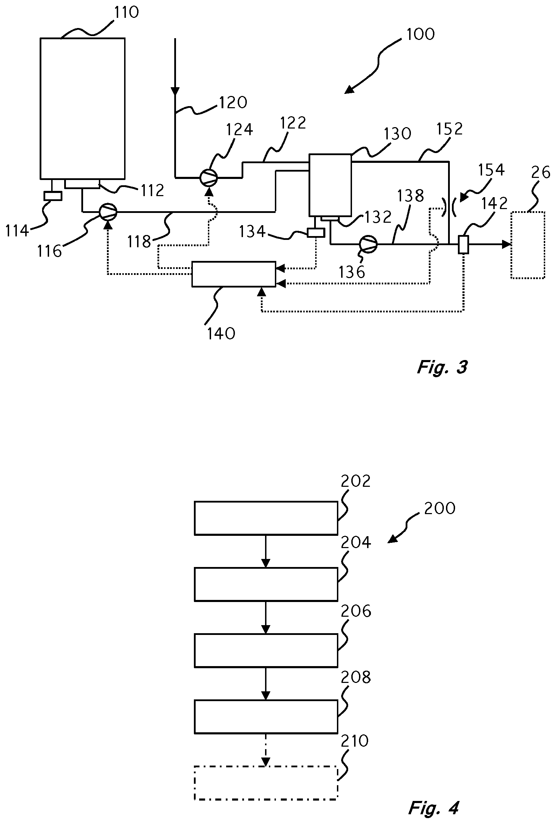

The hydrogen peroxide supply system 100 will from hereon be described with reference to Figs. 2 and 3. The hydrogen peroxide supply system 100 is not exclusively intended for the machine type described with reference to Fig. 1, but for all fill machines utilizing hydrogen peroxide vapor for sterilizing ready-to-fill packages.

The hydrogen peroxide supply system 100 is described more in detail in Fig. 2. It has a supply 110 for hydrogen peroxide. The supply 110 is configured to store hydrogen peroxide at a relatively high concentration, i.e. a concentration level being far higher than what is required for sterilization. The supply may e.g. store undiluted hydrogen peroxide provided by a supplier. For example, while a concentration of 2-5% is normally required for sterilization, the supply 110 may store hydrogen peroxide at a concentration of 30-40%. At such relatively high concentration, the breakdown of hydrogen peroxide to water and hydrogen is low meaning that the initial concentration level will remain the same for a long time without the need for changing to fresh hydrogen peroxide. The supply 110, which is preferably in the form of a tank, is provided with a discharge outlet 112 for allowing a flow of hydrogen peroxide out from the supply 110. In other embodiments the supply 110 may be a container and being provided with a suction line.

A sensor 114, e.g. in the form of a scale, may be provided for monitoring the current level of hydrogen peroxide inside the supply tank 110.

The supply 110 may also be provided with a discharge pump 116 drawing hydrogen peroxide out through the discharge outlet 112, and further downstream via a feeding line 118.

The system 100 further comprises a supply 120 of water. The water may preferably be de-ionized, filtered, or treated in any other suitable way for increasing the purity of the water, and to reduce the presence of microorganisms. The water supply 120 may e.g. be in the form of a fluid line 122 having a feeding pump 124 connected to it for feeding water downstream through the fluid line 122.

The hydrogen peroxide fluid line 118 and the water fluid line 122 are both connected to an intermediate storage 130. The intermediate storage 130, being in this embodiment realized as a tank or vessel, receives a flow of high concentration hydrogen peroxide and water. The purpose of the intermediate storage 130 is to mix the flows at a predetermined ratio in order to achieve a desired concentration of hydrogen peroxide for the hydrogen peroxide vapor generating system 26 of the fill machine 10.

The intermediate storage 130 is provided with a discharge outlet 132 at its lower end, and a sensor 134 may be provided for monitoring the current level inside the intermediate storage 130. The purpose of the intermediate storage 130 is not to keep diluted hydrogen peroxide for a long time, but instead to act as an in-line dilution chamber for immediately forwarding diluted hydrogen peroxide to the hydrogen peroxide vapor generating system 26 of the fill machine. This means that the intermediate storage 130 does not need to be particularly large in size, but an interior volume capable of enclosing up to 5-10 dl of diluted hydrogen peroxide will normally be sufficient to a large scale and high speed industrial fill machine.

The forward feeding of diluted hydrogen peroxide is realized by means of a pump 136 arranged in a fluid line 138 connecting the discharge outlet 132 of the intermediate storage 130 to downstream equipment, i.e. the hydrogen peroxide vapor generating system 26 forming an evaporator of the sterilization station 16. In some embodiments the pump 136 forms part of the downstream equipment.

The flow of hydrogen peroxide, here realized by means of the pump 136, is typically controlled by a control unit for the entire sterilization station 16, wherein the operating speed of the pump 136 is set to the current sterilization requirements (i.e. dependent on the amount of diluted hydrogen peroxide needed for sterilization).

However, the hydrogen peroxide supply system 100 also comprises a control unit 140, which may be a stand-along control unit or it may be integrated in the control unit of the entire sterilization station 16 (not shown). The control unit 140 is configured to control the operation of the pump 116 of the hydrogen peroxide supply 110 and the pump 124 of the water supply 120 in order to provide the desired dilution of hydrogen peroxide exiting the intermediate storage 130. Hence, the control unit 140 is adapted to regulate the flow ratio between the hydrogen peroxide and the water by transmitting suitable control signals to the respective pump 116, 124.

For achieving such control the control unit 140 receives current values of operational parameters. Such operational parameters include the current concentration of the diluted hydrogen peroxide downstream the intermediate storage 130. For this purpose a concentration sensor 142 is provided in the fluid line 138, and the sensor 142 is connected to the control unit 140 which thus receives the current concentration level continuously during operation, or at pre-set time intervals. Preferably, the control unit 140 also receives input from the level sensor 134 of the intermediate storage 130. If the current level inside the intermediate storage 130 is critical, either being too low for ensuring a sufficient flow of diluted hydrogen peroxide to the downstream equipment 26 or too high thus risking overflow of the intermediate storage 130, the control unit 140 may adjust the operational speed of the respective pumps 116, 124 accordingly. Should the measured concentration of the diluted hydrogen peroxide be within acceptable tolerances, the speed ratio of the pumps 116, 124 should be unchanged while the level inside the intermediate storage 130 is corrected. On the contrary, if the level inside the intermediate storage 130 is critical and needs correction, while the measured concentration of the diluted hydrogen peroxide is outside the predetermined thresholds, the speed ratio of the pumps 116, 124 may be changed during the control operation in order to solve both critical issues at the same time.

In order to improve mixing of water and hydrogen peroxide the hydrogen peroxide supply system 100 may be provided with a static mixer 150, arranged in the fluid line 138 upstream the concentration sensor 142.

In Fig. 3 another embodiment of a hydrogen peroxide supply system 100 is shown. The system 100 of Fig. 3 is almost identical to the embodiment described with reference to Fig. 2, except that the optional static mixer 150 is replaced by a recirculation line 152 feeding at least some of the discharged diluted hydrogen peroxide back to the intermediate storage 130. The amount of recirculated diluted hydrogen peroxide is preferably controlled by a flow restriction device 154, such as a flow control valve or similar. The flow restriction device 154 may either be static such that a fixed amount is always recirculated, or controllable in order to adapt the recirculation rate. For such embodiment, the controllable flow restriction device 154 may be connected to the control unit 140. Preferably, the recirculation line 152 is connected to the fluid line 138 upstream the concentration sensor 142.

Now turning to Fig. 4 a method 200 is schematically shown. The method 200 is performed in order to provide a flow of diluted hydrogen peroxide to a hydrogen peroxide vapor generating system 26 of a sterilization station 16 of a fill machine 10. The method 200 comprises a first step 202 of providing a first feed of hydrogen peroxide to an intermediate storage, a second step 204 of providing a second feed of water to said intermediate storage, a third step 206 of discharging diluted hydrogen peroxide from said intermediate storage, and a fourth step 208 of controlling the feeding rate of said first and second feeds in order to maintain a desired concentration of hydrogen peroxide in the discharge flow from said intermediate storage.

Optionally, the method 200 may also include a fifth step 210 of mixing the flow of diluted hydrogen peroxide, either by means of a static mixer or by means of a recirculation line back to the intermediate storage.

## Claims

1. A system (100) for hydrogen peroxide treatment, comprising
a supply (110) of hydrogen peroxide,
a supply (120) of water, and
an intermediate storage (130) for hydrogen peroxide being in fluid communication with the supply (110) of hydrogen peroxide and the supply (120) of water, wherein the system (100) further comprises a control unit (140) being connected to said supply (110) of hydrogen peroxide and the supply (120) of water for controlling the concentration of hydrogen peroxide in the intermediate storage (130).

2. The system (100) according to claim 1, wherein said supply (110) of hydrogen peroxide comprises a first pump (116) and said supply (120) of water comprises a second pump (124), and wherein said control unit (140) is connected to said pumps (116, 124).

3. The system (100) according to claim 1 or 2, wherein the intermediate storage (130) comprises a level sensor (134) for determining the fill level of said intermediate storage (130), and wherein the control unit (140) is configured to control the supply (110) of hydrogen peroxide and/or the supply (120) of water based on the detected fill level.

4. The system (100) according to any one of the preceding claims, further comprising a sensor (142) for detecting the concentration of hydrogen peroxide downstream the intermediate storage (130), and wherein the control unit (140) is configured to control the supply (110) of hydrogen peroxide and/or the supply (120) of water based on the detected concentration of hydrogen peroxide..

5. The system (100) according to claim 4, wherein said sensor (142) for detecting the concentration of hydrogen peroxide is arranged in fluid communication with an outlet of the intermediate storage (132).

6. The system (100) according to any one of the preceding claims 2-5, wherein said intermediate storage (130) comprises a third pump (136) for feeding diluted hydrogen peroxide from said intermediate storage (130) to a downstream evaporator (26) of a filling machine (10).

7. The system (100) according to any one of the preceding claims, further comprising mixing means (150, 152, 154) arranged downstream the intermediate storage (130).

8. The system (100) according to claim 7, wherein said mixing means (150, 152, 154) is a recirculation line (152) being provided with a flow restriction device (154) arranged to recirculate at least some of the diluted hydrogen peroxide exiting the intermediate storage (130) back to said intermediate storage (130).

9. The system (100) according to claim 7, wherein said mixing means (150, 152, 154) is a static mixer (150).

10. A method for providing a flow of hydrogen peroxide, comprising:
providing a first feed of hydrogen peroxide to an intermediate storage,
providing a second feed of water to said intermediate storage, and
discharging diluted hydrogen peroxide from said intermediate storage, wherein the method further comprises
controlling the feeding rate of said first and second feeds in order to maintain a desired concentration of hydrogen peroxide in the discharge flow from said intermediate storage.
